# EUROPEAN PATENT APPLICATION

(11) **EP 2 799 113 A1**
(43) Date of publication of application: **05.11.2014**
(21) Application number: 11879148.2
(22) Date of filing: 29.12.2011
(51) Int. Cl.: A61N 7/00, A61B 18/04, A61B 8/08

(54) **METHOD USING TRANSMITTED AND RECEIVED SIGNALS FOR FORMING ULTRASONIC IMAGES FOR ULTRASONIC DIAGNOSIS, AND HIGH INTENSITY FOCUSED ULTRASONIC THERAPEUTIC DEVICE PERFORMING SAME**

(71) Applicant: Alpinion Medical Systems Co., Ltd., Hwaseong-si, Gyeonggi-do 445-380 (KR)
(72) Inventor: KIM, Taeho, Seoul 136-130 (KR); SON, Keonho, Seongnam Si Gyeonggi-Do 463-970 (KR)
(74) Representative: Thorniley, Peter
(86) International application number: PCT/KR2011/010334
(87) International publication number: WO 2013/100232

(57) **Abstract**

The present disclosure relates to a method for forming ultrasonic images using transmitted and received signals for ultrasonic diagnosis and a HIFU therapeutic device performing the same. The HIFU therapeutic device includes a transceiver for transmitting a diagnostic ultrasound to a subject, and receive a reflected subject ultrasonic echo signal to form a received signal; an image processor for forming a B-mode image based on the received signal and output the B-mode image through a display unit; an ultrasonic wave generator for transmitting a high intensity ultrasound to a specific area of the subject; and a control unit for enabling the transceiver and the ultrasonic wave generator to control transmission periods of the diagnostic ultrasound and the high-intensity ultrasound wave based on information on at least one of a prescribed HIFU PRF (Pulse Repetition Frequency) and duty ratio and a PRF disable signal generated by the ultrasonic wave generator.

## Description

### [Technical Field]

The present disclosure in some embodiments relates to a method for forming ultrasonic images by using transmitted and received signals for ultrasonic diagnosis and a high-intensity focused ultrasound therapeutic device for the same. More particularly, the present disclosure relates to a method for forming ultrasonic images by using transmitted and received signals for ultrasonic diagnosis and a high-intensity focused ultrasound therapeutic device, capable of identifying a subject requiring ultrasonic therapy more precisely by cancelling an interference caused by a high-intensity ultrasound wave from an image formed of a diagnostic ultrasound when performing a high-intensity focused ultrasound therapy.

### [Background]

The statements in this section merely provide background information related to the present embodiment and may not constitute prior art.

A high-intensity focused ultrasound (HIFU) is generally used to cure (treat) tissues such as cancer, tumor and lesion. A form of treatment using a high-intensity ultrasound wave causes necrosis of a relevant body tissue by using heat occurring when the high-intensity ultrasound wave is focused at a point and transmitted thereto. To this end, the high-intensity ultrasound wave is duly controlled to prevent healthy tissues from being damaged, whereas the treatment using the high-intensity ultrasound wave can obviate an incision occurring when performing a surgical operation.

A conventional HIFU treatment method is performed by transmitting ultrasonic wave to a body tissue to be treated, acquiring an image by using an echo signal reflected at the relevant body tissue, and performing a HIFU based detection of changes in the relevant body tissue. Herein, an interference caused by the high-intensity ultrasound wave affects the image, thereby disturbing a correct presentation of the relevant image.

### [Disclosure]

### [Technical Problem]

The present disclosure provides a method for forming ultrasonic images by using transmitted and received signals for ultrasonic diagnosis and a HIFU therapeutic device for the same, capable of identifying a subject requiring ultrasonic therapy more precisely by cancelling an interference caused by a high-intensity ultrasound wave from an image formed of a diagnostic ultrasound when performing a HIFU therapy.

### [Summary]

According to an aspect of the present disclosure, there is provided a HIFU therapeutic device, including a transceiver, an image processor, an ultrasonic wave generator and a control unit. The transceiver is configured to transmit a diagnostic ultrasound to a subject, and receive an ultrasonic echo signal reflected from the subject to form a received signal. The image processor is configured to form a B-mode image based on the received signal, and output the B-mode image on a display unit. The ultrasonic wave generator is configured to transmit a high-intensity ultrasound wave to a specific area of the subject. And the control unit is configured to enable the transceiver and the ultrasonic wave generator to control transmission periods of the diagnostic ultrasound and the high-intensity ultrasound wave based on information on at least one of a prescribed HIFU PRF (Pulse Repetition Frequency) and a prescribed duty ratio and a PRF disable signal generated by the ultrasonic wave generator.

Further, according to another aspect of the present disclosure, there is provided a method for forming ultrasonic images using transmitted and received signals for ultrasonic diagnosis, including transmitting, by using a transceiver, a diagnostic ultrasound to a subject and receiving an ultrasonic echo signal reflected from the subject to form a received signal; forming, by using an image processor, a B-mode image based on the received signal and outputting the B-mode image on a display unit; transmitting, by using an ultrasonic wave generator, a high-intensity ultrasound wave to a specific area of the subject; and controlling, by using a control unit, the transceiver and the ultrasonic wave generator to control transmission periods of the diagnostic ultrasound and the high-intensity ultrasound wave based on information on at least one of a prescribed HIFU PRF (Pulse Repetition Frequency) and a prescribed duty and a PRF disable signal generated by the ultrasonic wave generator.

### [Advantageous Effects]

According to the present embodiment, a treatment effect can be maximized by making a subject requiring ultrasonic therapy presented more correctly by cancelling an interference caused by a high-intensity ultrasound wave from an image formed of a diagnostic ultrasound when performing a HIFU therapy.

Moreover, a treatment region can be correctly located during a HIFU treatment, resulting in the effective treatment capable of ensuring patient's stability and reducing the HIFU treatment time.

### [Description of Drawings]

FIG. 1 is a schematic block diagram of a HIFU therapeutic device according to the present embodiment.
FIG. 2 is a flowchart of a method for forming an ultrasonic image based on a HIFU PRF and a duty ratio according to a first embodiment.
FIG. 3 is a flowchart of a method for forming an ultrasonic image based on a PRF disable signal according to a second embodiment.
FIG. 4 is an exemplary diagram of forming an ultrasonic image based on a HIFU PRF and a duty ratio according to a first embodiment.
FIG. 5 is an exemplary diagram of forming an ultrasonic image based on a PRF disable signal according to a second embodiment.
FIG. 6 is an exemplary diagram illustrating an image free from interference according to the present embodiment.

### [Detailed Description]

Hereinafter, a detailed description of at least one embodiment is given with reference to accompanying drawings.

A high-intensity ultrasound wave described in the present embodiment refers to an ultrasound whose intensity is about 100 thousand times stronger than a diagnostic ultrasound wave. Further, a HIFU therapy described in the present embodiment refers to a procedure to burn out tissues of a specific area using heat of 64°C to 100°C occurring at a specific area by focusing a high-intensity ultrasound wave at a point (a specific area) and transmitting it thereto. When focusing a high-intensity ultrasound wave at a point (a specific area), whose intensity about 100 thousand times stronger than a regular diagnostic ultrasound, a heat is generated at a focal point. This is based on a similar principle similar to where a heat is generated at a focal point where sunlight is collected through a convex lens. Since ultrasound wave is harmless per se to human bodies and generates heat at a confined focal point where ultrasound is concentrated, ultrasound therapeutic method has been used to cure lesions in a human body and obviates the need to use knife or needle nor putting a person under general anesthesia.

Further, a B-mode image described in the present embodiment is a grayscale image which refers to an image mode indicating movement of a subject, and a C-mode image refers to a color flow image mode. Meanwhile, a BC-mode image refers to an image mode which indicates flow of blood or movement of a subject using Doppler Effect providing a B-mode image and a C-mode image simultaneously. This image mode provides information on flow of blood and movement of a subject together with anatomical information. In other words, the B-mode is a grayscale image which refers to an image mode indicating movement of a subject, and the C-mode is a color flow image which refers to an image mode indicating flow of blood or movement of a subject. Meanwhile, a HIFU device 100 described in the present disclosure can provide the B-mode image and the C-mode image, simultaneously. For the purpose of illustration, the present disclosure assumes that a HIFU therapeutic device 100 provides a B-mode image.

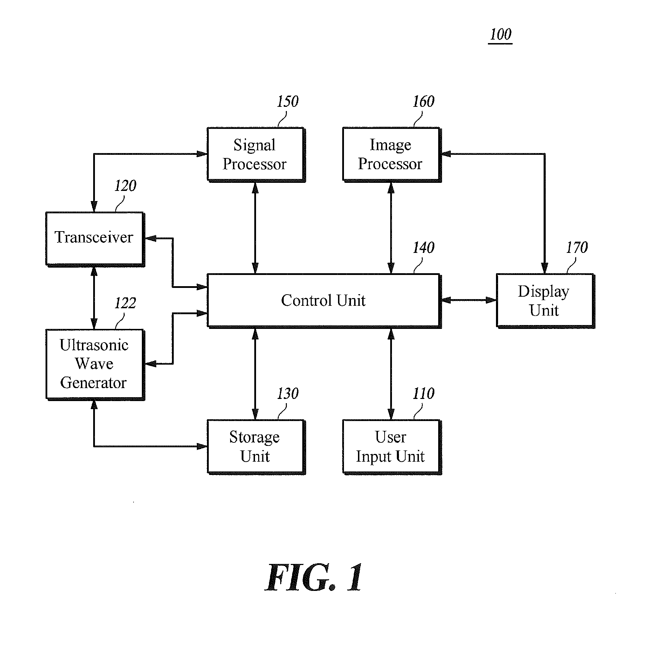

FIG. 1 is a schematic block diagram of a HIFU therapeutic device according to the present embodiment.

The HIFU therapeutic device 100 according to the present embodiment includes a user input unit 110, a transceiver 120, an ultrasonic wave generator 122, a storage unit 130, a control unit 140, a signal processor 150, an image processor 160 and a display unit 170. Meanwhile, although the present embodiment is described to include only the user input unit 110, transceiver 120, ultrasonic wave generator 122, storage unit 130, control unit 140, signal processor 150, image processor 160 and display unit 170, it should be noted that the above described construction is merely exemplary for the technical idea of the present embodiment and those skilled in the art can make a variety of modifications and variations to the constituents included in the HIFU therapeutic device 100, if not departing from intrinsic characteristics of the present embodiment.

The user input unit 110 receives instructions inputted by a user's manipulation. Here, the user instructions may be setting instructions to control the HIFU therapeutic device 100.

The transceiver 120 transmits a diagnostic ultrasound wave to a subject and receives an ultrasonic echo signal reflected from the subject as a received signal. In other words, the transceiver 120 transmits a diagnostic ultrasound used to obtain a B-mode image (or a C-mode image) to the subject and receives an ultrasonic echo signal reflected from the subject as a received signal. Further, the transceiver 120 transmits a diagnostic ultrasound to a subject and receives an ultrasonic echo signal reflected from the subject, based on a control signal received from the control unit 140, to form a received signal. Further, the transceiver 120 transmits and receives an ultrasound at a PRF (Pulse Repetition Frequency) to and from an area of interest, based on a control signal received from the control unit 140, to form a received signal. Here, the received signal includes a Doppler signal and a clutter signal. The Doppler signal is an ultrasonic signal that is originated from the transceiver 120 and then reflected by flowing blood, which has a relatively high frequency but has a relatively low intensity. The clutter signal is an ultrasonic signal that is originated from the transceiver 120 and then reflected by a cardiac wall, a cardiac plate, and so on, which has a relatively low frequency but has a relatively high intensity.

The transceiver 120 includes a probe (not shown) that serves to transmit and receive an ultrasound and a beam former (not shown) that serves to make transmit focusing and receive focusing of an ultrasound. Here, the probe includes a plurality of 1 dimensional or 2 dimensional array transducers. The probe transmits focused ultrasonic beam to a subject (not shown) along a transmission scan line by suitably delaying input time of pulses that are fed into each transducer. Meanwhile, the ultrasonic echo signal reflected from the subject is fed into each transducer with different reception time and each transducer outputs the input ultrasonic echo signal to the beam former. The beam former focuses the ultrasound to a specific position by manipulating a drive timing of each transducer in the probe when the probe transmits the ultrasound. The beam former focuses the ultrasonic echo signal by allocating time delay to each ultrasonic echo signal of the probe considering that the ultrasonic echo signals reflected by the subject arrive at the respective transducers of the probe at different times.

The transceiver 120 basically transmits a diagnostic ultrasound to a subject and receives an ultrasonic echo signal reflected from the subject correspondingly to the diagnostic ultrasound to form a received signal. Herein, the transceiver 120 according to a first embodiment performs the transmission and reception and further receives a second ultrasonic echo signal reflecting the switching period of switching into a disable state for the diagnostic ultrasound and the switching period of switching into an enable state, to form a second received signal. Meanwhile, when the transceiver 120 according to a second embodiment receives an ultrasonic echo signal corresponding to the diagnostic ultrasound transmitted to the subject, it receives a third ultrasonic echo signal reflecting the period of switching into a disable state according to a PRF disable signal and the period of switching into an enable state in order to form a third received signal.

The ultrasonic wave generator 122 transmits a high-intensity ultrasound wave to a specific area of a subject. In particular, the ultrasonic wave generator 122 transmits the high-intensity ultrasound wave to a specific location designated by the user input unit 110. Here, the user first transmits a diagnostic ultrasound to a subject using the transceiver 120, and then determines a specific area of the subject from an image generated based on a received signal that is formed by receiving an ultrasonic echo signal reflected from the subject. Here, in order for the user to determine the specific area, he or she can do it by inputting a value of the position corresponding to the specific area or manipulating a joystick-shaped navigation key. This input or manipulation allows transmitting the high-intensity ultrasound wave to a specific area of subjects such as cancer tissue, tumor tissue and lesion tissue. Here, the ultrasonic wave generator 122 may be manufactured in a circular shape, and preferably be embodied to have the transceiver 120 in its center, though it is not limited thereto.

The ultrasonic wave generator 122 according to the first embodiment transmits a high-intensity ultrasound wave to the specific area of the subject under the control of the control unit 140 based on a prescribed duty ratio. The ultrasonic wave generator 122 receives a trigger signal transmitted by the control unit 140, and transmits the high-intensity ultrasound wave based on the trigger signal. Meanwhile, the ultrasonic wave generator 122 according to the second embodiment generates a PRF disable signal when transmitting the high-intensity ultrasound wave, and transmits it to the transceiver 120 or the control unit 140. When transmission of the high-intensity ultrasound wave is stopped, the ultrasonic wave generator 122 according to the second embodiment stops transmitting the PRF disable signal. When a transmission command for the high-intensity ultrasound wave is inputted from the user input unit 110, the ultrasonic wave generator 122 transmits the high-intensity ultrasound wave to a specific area of the subject, and when a stop instruction of the high-intensity ultrasound wave is inputted from the user input unit 110, the ultrasonic wave generator 122 stops transmitting the high-intensity ultrasound wave.

The storage unit 130 stores a received signal formed through the transceiver 120. Further, the storage unit 130 stores information on a number of cut off frequencies to remove a clutter signal from the received signal.

The control unit 140 refers to a control means for controlling and coordinating the overall operation of the HIFU therapeutic device 100. The control unit 140 according to the present embodiment sets up a desired HIFU PRF and a duty ratio based on information inputted by the user input unit 110. Further, the control unit 140 according to the present embodiment enables the transceiver 120 and the ultrasonic wave generator 122 to control transmission period of a diagnostic ultrasound and a high-intensity ultrasound wave on the basis of information on at least one of the desired HIFU PRF and the duty ratio and a PRF disable signal generated through the ultrasonic wave generator 122.

The operational processes of the control unit 140 based on the prescribed PRF and the prescribed duty ratio according to the first embodiment will now be described in detail. The control unit 140 instructs the transceiver 120 to keep the transmission state of the diagnostic ultrasound in an enable state until the arrival of a transmission period of the high-intensity ultrasound wave in the ultrasonic wave generator 122 based on the set desired PRF and the duty ratio. Further, when the ultrasonic wave generator 122 transmits a high-intensity ultrasound wave as long as the prescribed duty ratio at a period corresponding to the set desired PRF, the control unit 140 according to the first embodiment instructs the transceiver 120 to switch the transmission state of the diagnostic ultrasound into a disable state at a period corresponding to the set desired PRF during the prescribed duty ratio. In this case, when the ultrasonic wave generator 122 stops transmitting the high-intensity ultrasound wave based on the prescribed duty ratio, the control unit 140 according to the first embodiment instructs the transceiver 120 to switch the transmission state of the diagnostic ultrasound into an enable state. Meanwhile, when the control unit 140 according to the first embodiment instructs the transceiver 120 to switch the transmission state of the diagnostic ultrasound to a disable state based on the prescribed duty ratio, it transmits a trigger signal to the ultrasonic wave generator 122 so that the ultrasonic wave generator 122 can transmit a high-intensity ultrasound wave.

The operational processes of the control unit 140 based on the PRF disable signal according to the second embodiment will now be described in detail. While receiving the PRF disable signal from the ultrasonic wave generator 122, the control unit 140 instructs the transceiver 120 to switch the transmission state of the diagnostic ultrasound into a disable state. Then, while receiving no PRF disable signal from the ultrasonic wave generator 122, the control unit 140 according to the second embodiment instructs the transceiver 120 to switch the transmission state of the diagnostic ultrasound into an enable state.

When receiving area of interest setup information inputted from the user input unit 110, the control unit 140 uses the information to control transmission and reception of ultrasound. Further, the control unit 140 performs appropriate control operations for repeatedly performing transmission and reception of the ultrasound for obtaining a B-mode image and transmission and reception of the ultrasound for obtaining a C-mode image.

The signal processor 150 performs a clutter filtering for a signal received from the transceiver 120 by setting a number of filters that have cut off frequencies to remove a clutter signal for each pixel within the area of interest. Meanwhile, the signal processor 150 may perform signal processing such as a gain control for image optimization with respect to the received signal from the transceiver 120. Further, the signal processor 150 performs a low pass filtering of the received signal and then transmits the filtered signal to the image processor 160.

The image processor 160 forms a B-mode image or a C-mode image based on the received signal, and outputs the B-mode image and the C-mode image on a display unit 170. Basically, the image processor 160 forms the B-mode image based on the received signal and outputs the B-mode image through the display unit 170.

In this case, the image processor 160 according to the first embodiment forms the B-mode image free from interference due to the high-intensity ultrasound wave, based on the second received signal formed by the transceiver 120, and outputs the B-mode image through the display unit 170. Meanwhile, the image processor 160 according to the second embodiment forms a B-mode image from which interference caused by a high-intensity ultrasound wave is removed based on a third received signal, and outputs the B-mode image through the display unit 170.

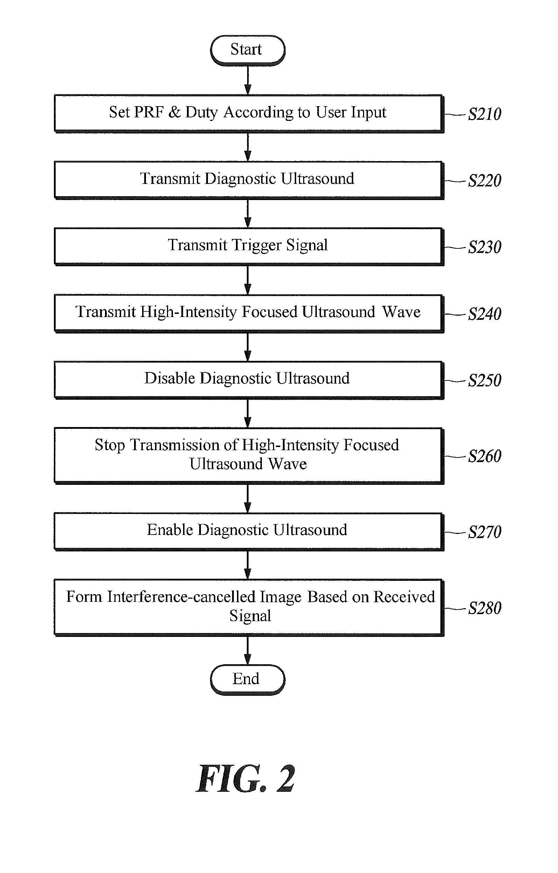

FIG. 2 is a flowchart of a method for forming an ultrasonic image based on a HIFU PRF and a duty ratio according to a first embodiment.

The control unit 140 included in the HIFU therapeutic device 100 sets up a prescribed HIFU PRF or a prescribed duty ratio based on information inputted by the user input unit 110 at step S210. A transceiver 120 in the HIFU therapeutic device 100 transmits a diagnostic ultrasound to a subject and receives an ultrasonic echo signal reflected from the subject to form a received signal (S220).

The control unit 140 of the HIFU therapeutic device 100 transmits a trigger signal to the ultrasonic wave generator 122 based on the prescribed duty ratio (S230), so that the ultrasonic wave generator 122 transmits a high-intensity ultrasound wave (S240). In other words, when the control unit 140 instructs the transceiver 120 to switch the transmission state of the diagnostic ultrasound into a disable state at step S230, the control unit 140 transmits a trigger signal to the ultrasonic wave generator 122. Further, the image processor 160 included in the HIFU therapeutic device 100 forms a B-mode image (or a C-mode image) based on a received signal at step 230, and outputs the B-mode image (the C-mode image) through the display unit 170. Meanwhile, the ultrasonic wave generator 122 in the HIFU therapeutic device 100 transmits a high-intensity ultrasound wave to a specific area of the subject at step S240.

After step S240, the control unit 140 of the HIFU therapeutic device 100 controls the transceiver 120 and the ultrasonic wave generator 122 based on the prescribed duty ratio so that transmission periods of the diagnostic ultrasound and the high-intensity ultrasound wave are adjusted. First, the ultrasonic wave generator 122 of the HIFU therapeutic device 100 controls the transceiver 120 based on the prescribed duty ratio until the high-intensity ultrasound wave is transmitted, so that the transmission state of the diagnostic ultrasound maintains an enable state.

The control unit 140 transmits a trigger signal to the ultrasonic wave generator 122 based on the prescribed duty ratio so that the ultrasonic wave generator 122 transmits a high-intensity ultrasound wave, and allows the transceiver 120 to switch transmission state of the diagnostic ultrasound into a disable state (S250). The ultrasonic wave generator 122 stops transmitting the high-intensity ultrasound wave based on the prescribed duty ratio (S260). When the ultrasonic wave generator 122 stops transmitting the high-intensity ultrasound wave based on the prescribed duty ratio, the control unit 140 instructs the transceiver 120 to switch the transmission state of the diagnostic ultrasound into an enable state (S270).

Through the process up to step 270, the transceiver 120 of the HIFU therapeutic device 100 receives an ultrasonic echo signal corresponding to the transmitted diagnostic ultrasound as a second ultrasonic echo signal reflecting the switching period of switching into a disable state for the diagnostic ultrasound and the period of switching into an enable state, to form a second received signal. In this case, the image processor 160 of the HIFU therapeutic device 100 forms a B-mode image (or a C-mode image) free from interference due to a high-intensity ultrasound wave, based on the second received signal, and outputs the B-mode image (or the C-mode image) through the provided display 170 (S280). After step S280, the HIFU therapeutic device 100 may repeatedly perform steps S210 to S280 according to the prescribed duty ratio.

Although it is described that steps S210 to S280 are sequentially performed in FIG. 2, the above steps are merely an exemplary description of the technical idea of the present embodiment and those skilled in the art can make a variety of modifications and variations to the steps by changing and executing steps described in FIG. 2 or executing at least one of steps S210 to S280 in parallel, if not departing from intrinsic characteristics of the present embodiment and the steps in FIG. 2 are not limited to the sequential order.

As described above, a HIFU control method using transmitted and received signals for ultrasonic diagnosis according to the present embodiment described in FIG. 2 can be recorded in a record medium that can be embodied in program and read by computer. The computer readable record medium that has program recorded to implement a HIFU control method using transmitted and received signals for ultrasonic diagnosis according to the present embodiment, includes all kinds of record device in which computer system readable data are stored. Examples of such computer readable record medium include ROM, RAM, CD-ROM, magnetic tape, floppy disk, optical data storage device, etc. which may also include media in a form of carrier wave (for example, transmission through Internet). Further, the computer readable record medium may be distributed to computer systems connected with one another by a network, and may store and execute computer readable codes in a distributed system. Further, functional program, code and code segments to implement the present embodiment may be easily inferred by programmers skilled in the technical field of the present embodiment.

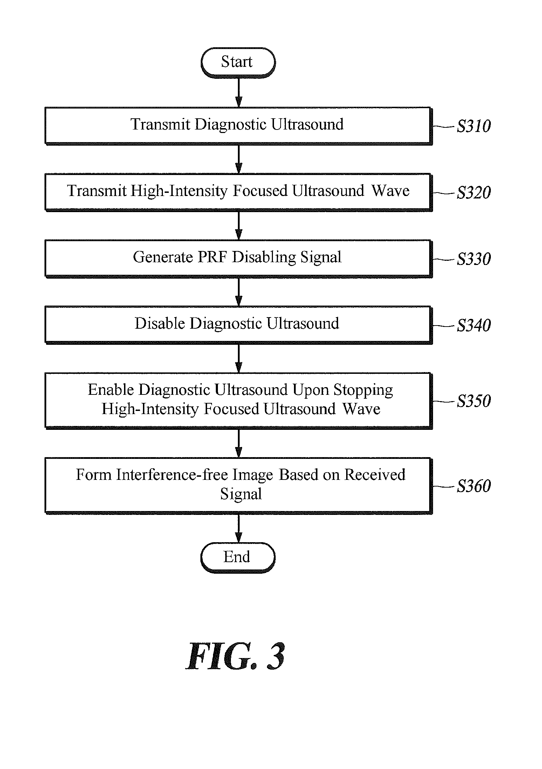

FIG. 3 is a flowchart of a method for forming an ultrasonic image based on a PRF disable signal according to a second embodiment.

The transceiver 120 included in the HIFU therapeutic device 100 transmits a diagnostic ultrasound to a subject and receives an ultrasonic echo signal reflected from the subject to form a received signal (S310). When there is a command to transmit a high-intensity ultrasound wave from the user input unit 110, the ultrasonic wave generator 122 included in the HIFU therapeutic device 100 transmits the high-intensity ultrasound wave to a specific area of the body (S320).

The ultrasonic wave generator 122 generates and sends a PRF disable signal to the transceiver 120 or the control unit 140 when transmitting the high-intensity ultrasound wave (S330). While receiving the PRF disable signal from the ultrasonic wave generator 122, the control unit 140 instructs the transceiver 120 to switch the transmission state of the diagnostic ultrasound into a disable state (S340). When there is a command to stop transmission of the high-intensity ultrasound wave through the user input unit 110, the ultrasonic wave generator 122 stops transmitting the high-intensity ultrasound wave. Further, while receiving no PRF disable signal from the ultrasonic wave generator 122, the control unit 140 makes the transceiver 120 switch the transmission state of the diagnostic ultrasound into an enable state (S350).

As receiving the ultrasonic echo signal corresponding to the diagnostic ultrasound transmitted to the subject, the transceiver 120 receives a third ultrasonic echo signal reflecting the period of switching into a disable state depending on the PRF disable signal and the period of switching into an enable state, to form a third received signal. Then, the image processor 160 included in the HIFU therapeutic device 100 forms a B-mode image (or a C-mode image) free from interference due to the high-intensity ultrasound wave, based on the third received signal, and outputs the B-mode image (or the C-mode image) through the display unit 170 (S360).

Although it is described that steps S310 to S350 are sequentially performed in FIG. 3, they are not limited to the sequential order since the above steps are merely an exemplary description of the technical idea of the present embodiment and those skilled in the technical field of the present embodiment can make a variety of modifications and variations to the steps by changing and executing steps described in FIG. 3 or executing at least one of steps S310 to S360 in parallel, if not departing from intrinsic characteristics of the present embodiment.

As described above, a method for forming ultrasonic images based on a PRF disable signal according to the second embodiment described in FIG. 3 can be recorded in a record medium that can be implemented in program and read by computer. The computer readable record medium that has program recorded to implement a method for forming an ultrasonic image based on a PRF disable signal according to the second embodiment includes all kinds of record devices in which computer system readable data are stored. Examples of such computer readable record medium include ROM, RAM, CD-ROM, magnetic tape, floppy disk, optical data storage device, etc. which may also include media implemented in a form of carrier wave (for example, transmission through Internet). Further, the computer readable record medium may be distributed to computer systems connected with one another by a network, and may store and execute computer readable codes in a distributed system. Further, functional program, code and code segments to implement the present embodiment may be easily inferred by programmers skilled in the technical field of the present embodiment.

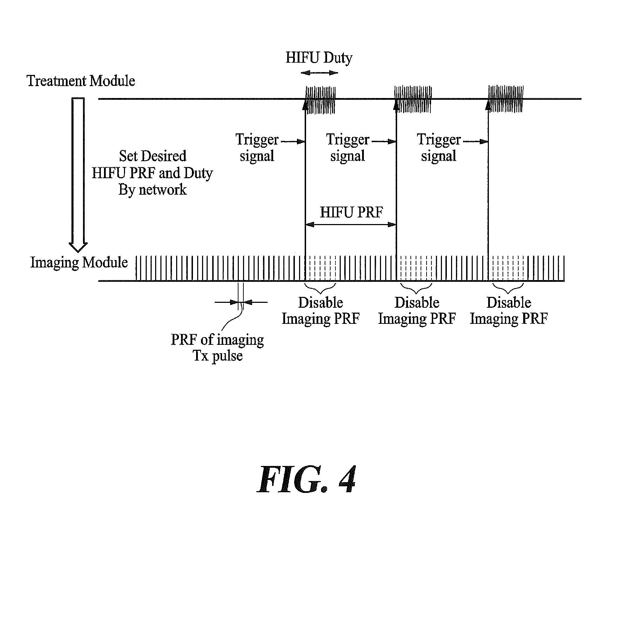

FIG. 4 is an exemplary diagram of forming an ultrasonic image based on a HIFU PRF and a duty ratio according to a first embodiment.

Brief definitions for respective modules shown in FIG. 4 will first be described. A 'treatment module' corresponds to the ultrasonic wave generator 122 in the present embodiment, and an 'imaging module' corresponds to the transceiver 120, the image processor 160 and the display unit 170 in the present embodiment combined. Here, the treatment module and imaging module may be operated under the control of the control unit 140 according to the present embodiment.

Referring to FIG. 4, the control unit 140 provides a prescribed HIFU PRF or a prescribed duty ratio in the treatment module and the imaging module based on information inputted through the user input unit 110.

The imaging module transmits a diagnostic ultrasound to a subject and receives an ultrasonic echo signal reflected from the subject to form a received signal.

Then, the imaging module transmits a trigger signal to the treatment module according to the prescribed duty ratio, in order to trigger the treatment module to transmit a high-intensity ultrasound wave. Here, the imaging module changes the transmission state of the diagnostic ultrasound into a disable state following the prescribed duty ratio. When the treatment module stops transmitting the high-intensity ultrasound wave according to the prescribed duty ratio, the imaging module changes the transmission state of the diagnostic ultrasound into an enable state.

Then, after a period of HIFU PRF, the imaging module transmits a trigger signal to the treatment module based on the prescribed duty ratio, so that the treatment module transmits a high-intensity ultrasound wave. In this case, the imaging module changes the transmission state of the diagnostic ultrasound into a disable state based on the prescribed duty ratio. When the treatment module stops transmitting a high-intensity ultrasound wave based on the prescribed duty, the imaging module switches the transmission state of the diagnostic ultrasound into an enable state. When such process is progressed, the imaging module receives an ultrasonic echo signal which corresponds to the transmitted subject diagnostic ultrasound as a second ultrasonic echo signal reflecting the period of switching into a disable state for the diagnostic ultrasound and the period of switching into an enable state, to form a second received signal. Then, the imaging module forms a B-mode image (or a C-mode image) free from interference due to the high-intensity ultrasound wave, and outputs the B-mode image (or the C-mode image) through a display unit 170.

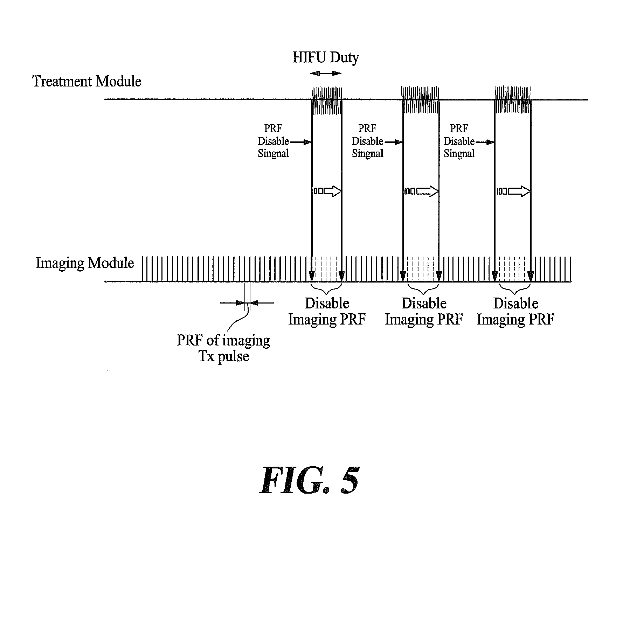

FIG. 5 is an exemplary diagram of forming an ultrasonic image based on a PRF disable signal according to a second embodiment.

To first define respective modules illustrated in FIG. 5, a 'treatment module' illustrated in FIG. 5 corresponds to the ultrasonic wave generator 122 in the present embodiment, and an 'imaging module' corresponds to the transceiver 120, the image processor 160 and the display unit 170 in the present embodiment combined. Here, the treatment module and the imaging module may operate under the control of the control unit 140 according to the present embodiment.

Referring to FIG. 5, the imaging module transmits a diagnostic ultrasound to a subject and receives an ultrasonic echo signal reflected from the subject to form a received signal. In this case, when the treatment module transmits a high-intensity ultrasound wave, it transmits a PRF disable signal to the imaging module. When receiving the PRF disable signal from the treatment module, the imaging module disables transmission of the diagnostic ultrasound. Further, when the treatment module stops to transmit the PRF disable signal, the 'imaging module' enables the transmission of the diagnostic ultrasound.

In particular, when such process develops, the imaging module receives an ultrasonic echo signal which corresponds to the transmitted subject diagnostic ultrasound as a third ultrasonic echo signal reflecting the period of switching into a disable state depending on the PRF disable signal and the period of switching into an enable state, to form a third received signal. Then, the imaging module forms a B-mode image (or a C-mode image) free from interference due to a high-intensity ultrasound wave, based on the third received signal, and outputs the B-mode image (or the C-mode image) through the display unit 170.

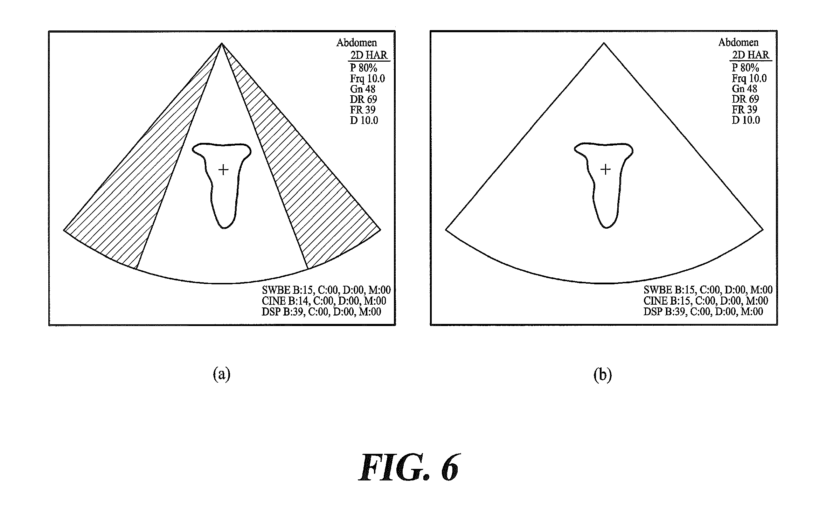

FIG. 6 is an exemplary diagram illustrating an interference-free image according to the present embodiment.

Referring to FIG. 6 at (a), the following will describe an ultrasonic echo signal reflecting neither of the period of switching into a disable state for a diagnostic ultrasound and the period of switching into an enable state. An 'imaging module' transmits a diagnostic ultrasound to a subject and receives an ultrasonic echo signal reflecting neither of the period of switching into a disable state for a diagnostic ultrasound and the period of switching into an enable state, to form a received signal. Then, the imaging module forms a B-mode image (or a C-mode image) free from interference due to a high-intensity ultrasound wave, based on the received signal, and outputs the B-mode image (or the C-mode image) through the display unit 170. Here, the image formed at this time shows an interference phenomenon as hatched sections in FIG. 6 at (a). Because of such an interference phenomenon, it becomes difficult to present a relevant image correctly when performing a HIFU therapy.

By comparison, as illustrated in FIG. 6 at (b), the imaging module receives an ultrasonic echo signal which corresponds to a transmitted subject diagnostic ultrasound as a second ultrasonic echo signal reflecting the period of switching into a disable state for the diagnostic ultrasound and the period of switching into an enable state, to form a second received signal. Then, the imaging module forms a B-mode image (or a C-mode image) free from interference due to a high-intensity ultrasound wave, based on the second received signal, and outputs the B-mode image (or the C-mode image) through the display unit 170. Therefore, a relevant image can be correctly presented when performing a HIFU therapy since the B-mode image (or the C-mode imager) is formed interference free.

Meanwhile, referring to FIG. 6 at (b), the imaging module receives an ultrasonic echo signal which corresponds to a diagnostic ultrasound as a third ultrasonic echo signal reflecting the period of switching into a disable state depending on a PRF disable signal and the period of switching into an enable state, to form a third received signal. Then, the imaging module forms a B-mode image (or a C-mode image) free from interference due to a high-intensity ultrasound wave, based on the third received signal, and outputs the B-mode image (or the C-mode image) on the display unit 170. Hence, a relevant image can be correctly presented when performing a HIFU therapy since the B-mode image (or the C-mode imager) is formed interference free.

The above description is merely an exemplary description of the technical idea of the present disclosure, and those skilled in the art may make a variety of modifications and variations, if not departing from intrinsic characteristics of the present embodiment. Accordingly, the present embodiments are not aimed to restrict technical idea of the present disclosure but aimed to describe it, and the scope of the technical idea of the present disclosure is not restricted by the embodiments. The protective scope of the present disclosure should be construed by claims following below and all technical ideas within the equivalent scope should be construed to be included in the right scope of the present disclosure.

## Claims

1. A high-intensity focused ultrasound (HIFU) therapeutic device, comprising:
a transceiver configured to transmit a diagnostic ultrasound to a subject, and receive an ultrasonic echo signal reflected from the subject to form a received signal;
an image processor configured to form a B-mode image based on the received signal and output the B-mode image through a display unit;
an ultrasonic wave generator configured to transmit a high intensity ultrasound wave to a specific area of the subject; and
a control unit configured to enable the transceiver and the ultrasonic wave generator to control transmission periods of the diagnostic ultrasound wave and the high-intensity ultrasound wave based on an information on at least one of a prescribed HIFU PRF (Pulse Repetition Frequency) and a prescribed duty ratio and a PRF disable signal generated by the ultrasonic wave generator.

2. The HIFU therapeutic device of claim 1, wherein the control unit instructs the transceiver to maintain an enable state for a transmission state of the diagnostic ultrasound wave until the arrival of a transmission period of the high-intensity ultrasound wave in the ultrasonic wave generator according to the prescribed HIFU PRF and the prescribed duty ratio.

3. The HIFU therapeutic device of claim 2, wherein the control unit instructs the transceiver to switch the transmission state of the diagnostic ultrasound into a disable state during the prescribed duty ratio in a period corresponding to the set desired HIFU PRF, when the ultrasonic wave generator transmits the high-intensity ultrasound wave as much as the prescribed duty ratio in a period corresponding to the prescribed HIFU PRF.

4. The HIFU therapeutic device of claim 3, wherein the control unit instructs the transceiver to switch the transmission state of the diagnostic ultrasound into the enable state when the ultrasonic wave generator stops transmitting the high-intensity ultrasound wave based on the prescribed duty ratio.

5. The HIFU therapeutic device of claim 4, wherein the transceiver receives an ultrasonic echo signal which corresponds to the diagnostic ultrasound transmitted to the subject as a second ultrasonic echo signal reflecting a switching period of switching into the disable state for the diagnostic ultrasound and a period of switching into the enable state, to form a second received signal.

6. The HIFU therapeutic device of claim 5, wherein the image processor forms the B-mode image free from interference due to the high-intensity ultrasound wave, based on the second received signal, and outputs the B-mode image through the display unit.

7. The HIFU therapeutic device of claim 3, wherein the control unit transmits a trigger signal to the ultrasonic wave generator so that the ultrasonic wave generator transmits the high-intensity ultrasound wave, when instructing the transceiver to switch the transmission state of the diagnostic ultrasound into the disable state according to the prescribed duty ratio.

8. The HIFU therapeutic device of claim 1, wherein the ultrasonic wave generator generates a PRF disable signal when transmitting the high-intensity ultrasound wave and transmits the PRF disable signal to the transceiver or the control unit, and wherein the control unit instructs the transceiver to switch the transmission state of the diagnostic ultrasound into a disable state while the control unit receives the PRF disable signal.

9. The HIFU therapeutic device of claim 8, wherein the ultrasonic wave generator stops transmitting the PRF disable signal when the transmission of the high-intensity ultrasound wave is stopped, and wherein the control unit instructs the transceiver to switch the transmission state of the diagnostic ultrasound into an enable state while the control unit does not receive the PRF disable signal.

10. The HIFU therapeutic device of claim 9, wherein the transceiver receives an ultrasonic echo signal which corresponds to the diagnostic ultrasound transmitted to the subject as a third ultrasonic echo signal reflecting a switching period of switching into the disable state according to the PRF disable signal and a period of switching into the enable state, to form a third received signal.

11. The HIFU therapeutic device of claim 10, wherein the image processor forms the B-mode image free from interference due to the high-intensity ultrasound wave and outputs the B-mode image through the display unit.

12. The HIFU therapeutic device of claim 1, wherein the control unit sets the prescribed HIFU PRF and the prescribed duty ratio based on information inputted by a user input unit.

13. A method for forming ultrasonic images using transmitted and received signals for ultrasonic diagnosis, the method comprising:
transmitting, by using a transceiver, a diagnostic ultrasound to a subject and receiving an ultrasonic echo signal reflected from the subject to form a received signal;
forming, by using an image processor, a B-mode image based on the received signal and outputting the B-mode image through a display unit;
transmitting, by using an ultrasonic wave generator, a high-intensity ultrasound wave to a specific area of the subject; and
controlling, by using a control unit, the transceiver and the ultrasonic wave generator to control transmission periods of the diagnostic ultrasound and the high-intensity ultrasound wave based on an information on at least one of a prescribed HIFU PRF (Pulse Repetition Frequency) and a prescribed duty ratio and a PRF disable signal generated through the ultrasonic wave generator.

14. The method of claim 13, wherein the controlling comprises instructing, by the control unit, the transceiver to keep a transmission state of the diagnostic ultrasound into an enable state until the arrival of a transmission period of the high-intensity ultrasound wave in the ultrasonic wave generator based on the prescribed HIFU PRF and the prescribed duty ratio.

15. The method of claim 14, wherein the controlling comprises instructing, by the control unit, the transceiver to switch the transmission state of the diagnostic ultrasound into a disable state during the prescribed duty ratio in a period corresponding to the prescribed HIFU PRF, when the ultrasonic wave generator transmits the high-intensity ultrasound wave as much as the prescribed duty ratio in a period corresponding to the prescribed HIFU PRF.

16. The method of claim 15, wherein the controlling comprises instructing, by the control unit, the transceiver to switch the transmission state of the diagnostic ultrasound into the enable state when the ultrasonic wave generator stops transmitting the high-intensity ultrasound wave according to the prescribed duty ratio.

17. The method of claim 16, wherein the transmitting and receiving comprises receiving, by the transceiver, an ultrasonic echo signal which corresponds to the diagnostic ultrasound transmitted to the subject as a second ultrasonic echo signal reflecting a switching period of switching into the disable state for the diagnostic ultrasound and a period of switching into the enable state, to form a second received signal.

18. The method of claim 17, wherein the processing of the image comprises forming, by the image processor, the B-mode image free from interference due to the high-intensity ultrasound wave, based on the second received signal, and outputting the B-mode image through the display unit.

19. The method of claim 15, wherein the controlling comprises transmitting, by the control unit, a trigger signal to the ultrasonic wave generator so that the ultrasonic wave generator transmits the high-intensity ultrasound wave, when instructing the transceiver to switch the transmission state of the diagnostic ultrasound into the disable state according to the prescribed duty ratio.

20. The method of claim 13, wherein the controlling comprises:
generating, by using the ultrasonic wave generator, a PRF disable signal when transmitting the high-intensity ultrasound wave, and transmitting the PRF disable signal to the transceiver or the control unit; and
instructing, by using the control unit, the transceiver to switch the transmission state of the diagnostic ultrasound into a disable state while the control unit receives the PRF disable signal.

21. The method of claim 20, wherein the controlling comprises:
stopping, by the ultrasonic wave generator, to transmit the PRF disable signal when the transmission of the high-intensity ultrasound wave is stopped, and
instructing, by the control unit, the transceiver to switch the transmission state of the diagnostic ultrasound into an enable state while the control unit does not receive the PRF disable signal.

22. The method of claim 21, further comprising receiving, by the transceiver, an ultrasonic echo signal which corresponds to the diagnostic ultrasound transmitted to the subject as a third ultrasonic echo signal reflecting a switching period of switching into the disable state according to the PRF disable signal and a period of switching into the enable state, to form a third received signal.

23. The method of claim 22, further comprising forming, by the image processor, the B-mode image free from interference due to the high-intensity ultrasound wave and outputting the B-mode image through the display unit.

24. The method of claim 13, further comprising setting the prescribed HIFU PRF and the prescribed duty ratio based on information inputted by a user input unit.
